# EUROPEAN PATENT SPECIFICATION

(11) **EP 3 991 698 B1**
(45) Date of publication and mention of the grant of the patent: **20.12.2023**
(21) Application number: 21204410.1
(22) Date of filing: 25.10.2021
(51) Int. Cl.: A61F 2/86, A61F 2/954, A61F 2/06, A61B 17/11

(54) **MEDICAL IMPLANTS FOR ENHANCING THE HEALING RESPONSE AND THE EFFECTIVE LONGEVITY OF BLOOD VESSELS AND ANASTOMOSES**
MEDIZINISCHE IMPLANTATE ZUR VERBESSERUNG DER HEILUNGSREAKTION UND DER EFFEKTIVEN LANGLEBIGKEIT VON BLUTGEFÄSSEN UND ANASTOMOSEN
IMPLANTS MÉDICAUX POUR AMÉLIORER LA RÉPONSE DE CICATRISATION ET LA LONGÉVITÉ EFFICACE DE VAISSEAUX SANGUINS ET D'ANASTOMOSES

(30) Priority: 28.10.2020 US 202017082342
(43) Date of publication of application: 04.05.2022
(73) Proprietor: Mozarc Medical US LLC, Minneapolis, MN 55431 (US)
(72) Inventor: WOLF, Michael F., Minneapolis, CA 55432 (US); COLLIER, Kenneth J., Dellwood, MN 55110 (US); EPSTEIN, Evan D., Irvine, CA 92617 (US)
(74) Representative: Maschio, Antonio

(56) References cited:
- US-A1- 2008 305 999
- US-A1- 2013 041 453
- US-A1- 2016 000 985
- US-B1- 6 743 243
- US-B2- 7 892 246

## Description

### BACKGROUND

When performing hemodialysis, a vascular access device functions as an access point through which blood is removed and returned to the patient. The vascular access allows large amounts of blood to flow during hemodialysis treatments to filter as much blood as possible per treatment. An arteriovenous ("AV") fistula is one type of vascular access improvement and is a connection, made by a vascular surgeon, of an artery to a vein. An AV fistula causes added pressure and blood to flow into the vein making it grow and become stronger for easy and reliable access.

Coronary artery bypass grafting ("CABG") is a surgical procedure that uses a blood vessel from another part of the body, such as, for example, the great saphenous vein from the leg, and connects it to blood vessels above and below a narrowed or blocked coronary artery or arteries, thereby bypassing the narrowed or blocked coronary artery or arteries.

There is a need to provide stronger AV fistulas and coronary artery bypass grafts having less stressed venous tissue that allow for the natural expansion of the blood vessel(s) while also supporting the blood vessel(s).

US 2013/041453A1 discloses a medical implant for providing external support to an arteriovenous fistula.

### SUMMARY

In one aspect of the present disclosure, not covered by the invention, a method of establishing an anastomosis is provided and includes connecting a blood vessel and an artery, thereby forming an anastomosis therebetween; and wrapping an outer surface of the blood vessel with a first tubular support, whereby the first tubular support exerts a radially-inward force on the blood vessel.

In other aspects of the present disclosure, wrapping the outer surface of the blood vessel may include covering both a portion of the blood vessel and the anastomosis with the first tubular support.

In other aspects of the present disclosure, the method may further include positioning an end of the artery through an opening defined in a side of the first tubular support. The method may further include connecting the artery end to another blood vessel. The blood vessel may be a vein.

In other aspects of the present disclosure, the end of the artery may be positioned through the opening prior to connecting the vein and the artery and prior to wrapping the outer surface of the vein with the first tubular support.

In other aspects of the present disclosure, the method may further include wrapping an outer surface of the artery with a second tubular support.

In other aspects of the present disclosure, the method may further include connecting an end of the second tubular support with the side of the first tubular support.

In other aspects of the present disclosure, the method may further include positioning a segment of the blood vessel and a segment of the artery in parallel relation to one another. In aspects, the method may further include completing a vascular anastomosis of the artery and vein in the parallel region. The method may include wrapping the first tubular support around an outer surface of the connecting segment of the artery as the first tubular support is then also wrapped around the outer surface of the connecting segment of the vein.

In other aspects of the present disclosure, the method may further include ligating side branches from the blood vessel at a plurality of ligation sites; and aligning the ligation sites with respective open cells defined in the tubular support.

In other aspects of the present disclosure, wrapping the first tubular support may include transitioning the first tubular support from a substantially planar configuration to a cylindrical configuration. In the cylindrical configuration, a pair of longitudinal edges of the first tubular support may be disposed adjacent one another. Any degree of overlap between the longitudinal edges may be permitted to further strengthen the external support effect to limit the extent of radial expansion of an enclosed blood vessel.

In other aspects of the present disclosure, wrapping the first tubular support may include helically winding the first tubular support around the outer surface of the blood vessel. The first tubular support may surround the entire outer surface of the blood vessel or to any suitable extent needed to strengthen the external support effect.

The invention relates to an implant according to independent claim 1.

According to the invention, the main tubular body has a pair of longitudinal edges configured to be disposed adjacent one another when the main tubular body is in the closed configuration.

According to the invention, the pair of longitudinal edges together defines a seam that extends along a length of the main tubular body. The seam and the side opening may be disposed in opposing relation to one another.

In other aspects of the present disclosure, the side opening may have a circular shape. The circular shape may be a circle, an oval, or the like.

In other aspects of the present disclosure, the distal end portion of the main tubular body may have a distal edge that extends at a non-perpendicular angle relative to a longitudinal axis of the main tubular body.

In other aspects of the present disclosure, the medical implant may further include a branching tubular body configured to transition between an opened configuration and a closed configuration, in which the branching tubular body defines a longitudinally-extending branching passageway therethrough. The branching passageway may be in communication with the main passageway and may be configured to receive and support the second blood vessel. The branching tubular body may be resiliently biased toward the closed configuration and may include a branching proximal end portion and a branching distal end portion. The branching proximal end portion may define an opening in communication with the branching passageway and the branching distal end portion may define an opening in communication with the branching passageway.

In other aspects of the present disclosure, the main tubular body and the branching tubular body may be fabricated from a mesh that defines a plurality of discrete cells. Each of the discrete cells may have a smaller diameter than the side opening and may be sized to accommodate all or a portion of a ligated side branch on the exterior of the supported vein.

In other aspects of the present disclosure, the main tubular body and the branching tubular body may be fabricated from a shape memory material.

In accordance with another aspect of the disclosure, not covered by the invention, a method of establishing an arteriovenous fistula graft is provided and includes wrapping an outer surface of a vein with a tubular support; positioning an end of an artery through an opening defined in a side of the tubular support; and connecting the end of the artery to a side of the vein, thereby forming a fluid connection therebetween.

In other aspects of the present disclosure, the tubular support may define a longitudinally-extending passageway. The passageway may have an inner diameter that approximates an outer diameter of the vein or artery, whereby the tubular support exerts a radially-inward force on the vein or artery.

In other aspects of the present disclosure, wrapping the outer surface of the vein with the tubular support includes transitioning the tubular support from a substantially planar configuration to a cylindrical configuration. In the cylindrical configuration, a pair of longitudinal edges of the tubular support may be disposed adjacent one another. In other aspects, wrapping the outer surface of the vein with the tubular support may include helically winding the tubular support around the outer surface of the vein.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Embodiments of the present disclosure are described herein with reference to the accompanying drawings, wherein:
FIG. 1A is a perspective view illustrating an exemplary embodiment of a medical implant, shown in a closed configuration and with flat ends, for externally supporting an AV fistula or a CABG;
FIG. 1B is a perspective illustrating an exemplary embodiment of a medical implant, shown in a closed configuration with a 45 degree bevel on one end, for externally supporting an AV fistula or CABG;
FIG. 2 is a top view illustrating the medical implant of FIG. 1B shown in an opened configuration;
FIG. 3A is an enlarged perspective view of a flat end portion of the medical implant of FIG. 1A;
FIG. 3B is an enlarged perspective view of a beveled end portion of the medical implant of FIGS. 1B and 2;
FIG. 4A is an enlarged perspective view illustrating a longitudinal seam of the medical implant of FIG. 1A;
FIG. 4B is an enlarged end view illustrating the longitudinal seam of the medical implant of FIG. 4A;
FIG. 4C is an end view illustrating the longitudinal seam of the medical implant of FIG. 4A;
FIGS. 5A-5D are side cross-sectional views illustrating different degrees of overlap of longitudinal sides making up the longitudinal seam of the medical implant of FIG. 1A;
FIG. 6A is an enlarged perspective view illustrating an alternative embodiment of a medical implant for externally supporting an AV fistula and having an oval opening wherein an end-to-side anastomosis may be made to another vessel (e.g., a vein or an artery);
FIG. 6B is an enlarged perspective view illustrating another alternative embodiment of a medical implant for externally supporting an AV fistula and having a circular opening wherein an end-to-side anastomosis may be made to another vessel;
FIG. 7A is a side view illustrating another medical implant including the medical implant of FIG. 1B joined to a side of the medical implant of FIG. 6A;
FIG. 7B is a perspective view illustrating a medical implant assembly including separate first and second medical implants configured to be coupled to one another;
FIG. 7C is an enlarged view illustrating snapping hooks for securing medical implants to one another;
FIG. 8A is a perspective view illustrating another embodiment of a medical implant for externally supporting an AV fistula having a spiral slit;
FIGS. 8B and 8C are side views of medical implants with different cell densities;
FIG. 8D is a side view illustrating a medical implant wherein an end includes a solid ring for suturing;
FIG. 9A is a flow chart illustrating an exemplary method of utilizing the medical implants of the disclosure;
FIG. 9B is a flow chart illustrating another exemplary method of utilizing the medical implants of the disclosure;
FIG. 10 is a schematic illustration of a first type of vein-artery connection in an AV fistula or CABG;
FIG. 11 is a schematic illustration of a second type of vein-artery connection in an AV fistula;
FIG. 12 is a schematic illustration of a third type of vein-artery connection in an AV fistula;
FIG. 13 is a schematic illustration of a fourth type of vein-artery connection in an AV fistula;
FIG. 14 is an illustration of a coronary artery bypass graft surgery; and
FIG. 15 is a perspective view illustrating the medical implant of FIG. 1B having a spreading device disposed therein.

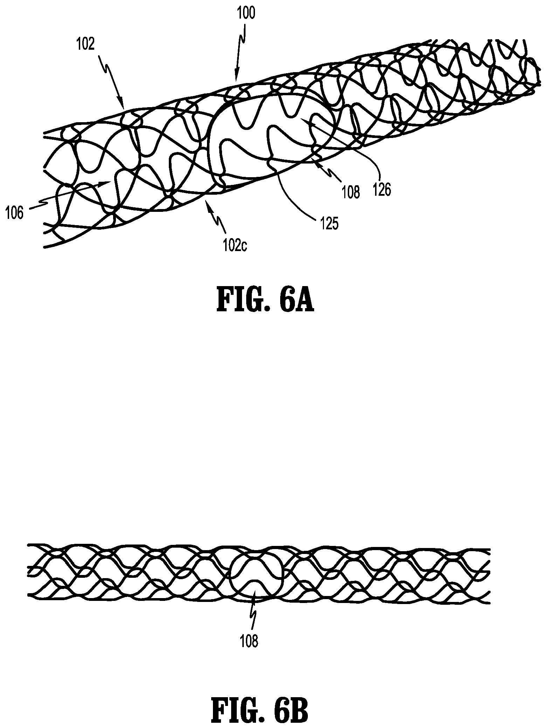

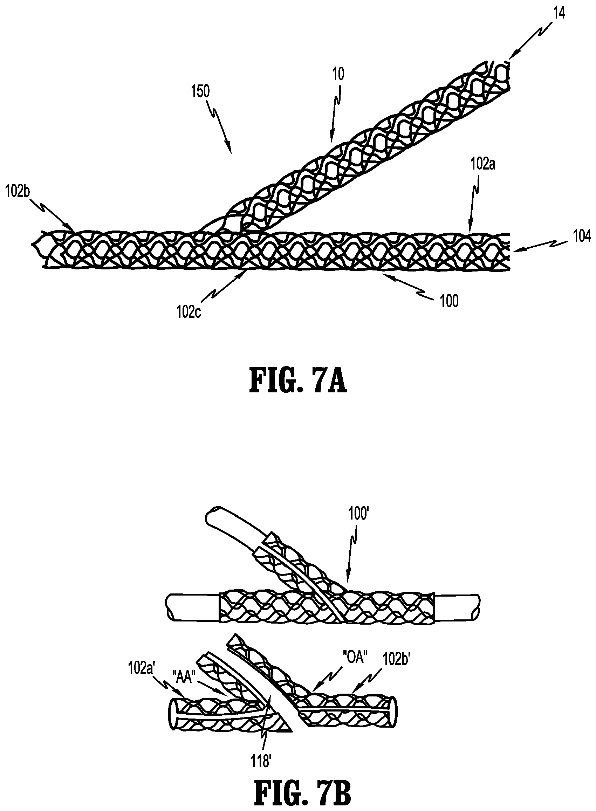

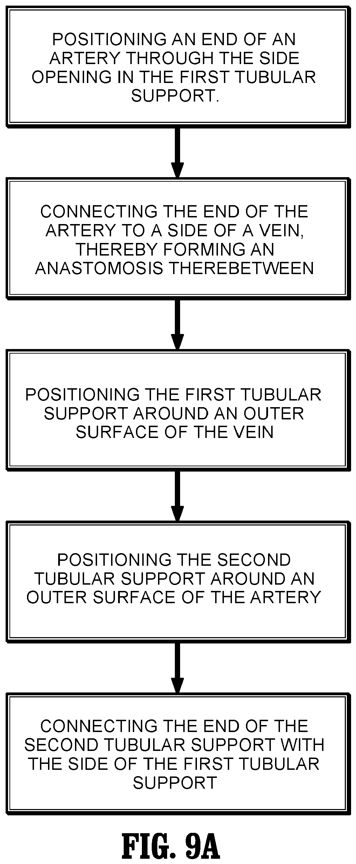

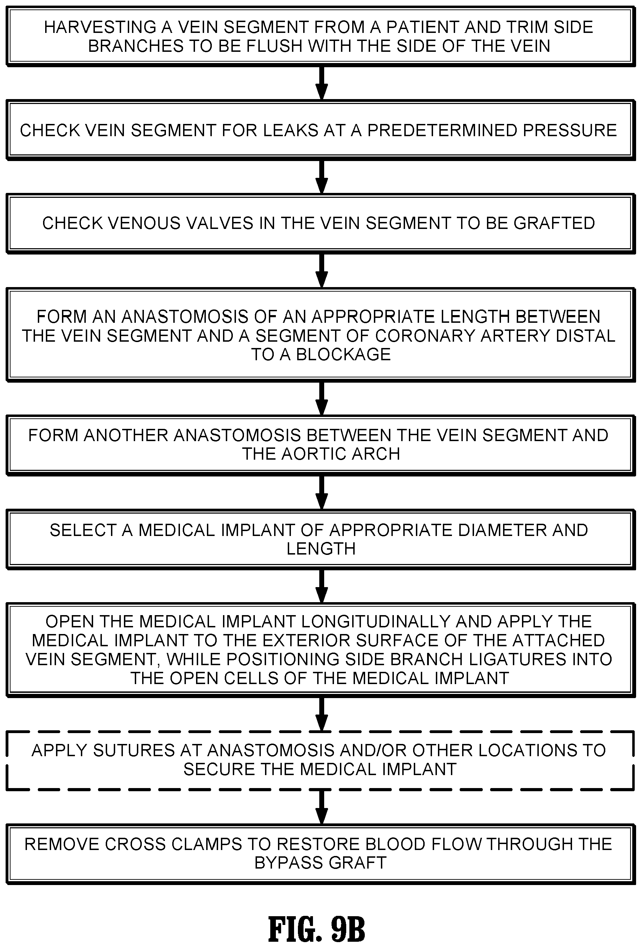

The invention relates to an implant as illustrated on fig. 6A, 6B and 7A. The methods described hereafter and illustrated on fig. 9A, 9B, 10-14 are not covered by the invention.

### DETAILED DESCRIPTION

Embodiments of the disclosed medical implants and methods are described in detail with reference to the drawings, in which like reference numerals designate identical or corresponding elements in each of the several views. As used herein the term "distal" refers to that portion of the medical implant, or component thereof, farther from the user, while the term "proximal" refers to that portion of the medical implant, or component thereof, closer to the user.

As will be described in detail below, a method of forming and supporting an arteriovenous ("AV") fistula graft is provided utilizing a tubular structure that surrounds a portion of a venous and/or arterial portion of the AV fistula. The tubular structure may be a mesh tube having a longitudinal seam with improved radial compliance and openings that mitigate the stenosis caused by side branch ligature. Further provided herein is a method of forming and supporting an autologous vein coronary artery bypass graft ("CABG") utilizing the tubular structure that surrounds the anastomosis between the coronary artery and the vein graft and/or the anastomosis between the aortic arch and the vein graft. Other features and benefits of the disclosed medical implants and methods of use thereof are further detailed below.

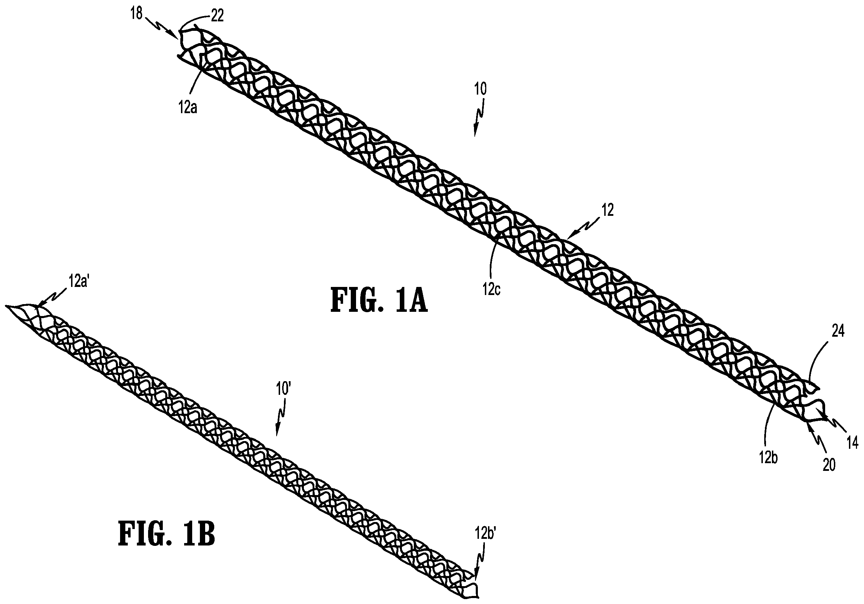

With reference to FIGS. 1A, 3A, and 4A, a medical implant, in accordance with an embodiment of the present disclosure, is generally designated as 10, and is in the form of a tubular support for providing external support to an AV fistula and/or a coronary artery bypass graft. The medical implant 10 includes a mesh-like tubular body 12 fabricated from a shape-memory material (e.g., nickel-titanium) configured to assume a cylindrical configuration, as shown in FIG. 1A. In aspects, the medical implant 10 may be self-expanding and formed from other suitable, biocompatible materials, such as, for example, shape-memory alloys, shape-memory polymers, steel, or cobalt-chromium.

In aspects, the medical implant 10 may be coated with or have embedded therein one or more therapeutic agents or delivery to a blood vessel after implantation. The therapeutic agent may be applied via dipping, ultrasonic spraying, electrostatic spraying, inkjet coating, and the like. The therapeutic agent may be capable of providing enhanced healing to the blood vessel and/or producing a beneficial effect against one or more conditions including coronary restenosis, cardiovascular restenosis, angiographic restenosis, arteriosclerosis, hyperplasia, and other diseases or conditions. The therapeutic agent may include, but are not limited to, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, an organic drug, a pharmaceutical compound, a recombinant DNA product, a recombinant RNA product, a collagen, a collagenic derivative, a protein, a protein analog, a saccharide, a saccharide derivative, any suitable pharmaceutical drug or therapeutic substance, or a combination thereof. In certain aspects, the medical implant 10 may also be coated with a polymer for improved biocompatibility. The polymer coating may or may not be biodegradable. In embodiments, the polymer coating may include the therapeutic agent.

The medical implant 10 may be machined or laser cut from a solid tube of material to form the interconnected strands according to the present disclosure. In other aspects, the medical implant 10 may formed by braiding metal wire, polymer filaments, or combinations thereof, into desired shapes described in the disclosure. In aspects, the medical implant 10 may be laser cut from a flat metal sheet. To get the medical implants 10 in the desired tube form, the implants 10 are heat set while wrapped around a mandrel. When the mandrel has the form of a straight tube, and the laser cut device is wrapped around the mandrel and then heat set. The result is a perfect tubular-shaped device with parallel longitudinal seams. When the mandrel has different diameters at either end, i.e., a long tapered conical-shaped mandrel, and the device is wrapped around the mandrel and heat set, the resultant device maintains the shape of a tapered cone with non-parallel seams and different diameters at either end.

The medical implant 10 may be configured to transition, either manually or automatically, between an opened configuration and the closed configuration (FIG. 1A). In the opened configuration, the medical implant 10 may assume a substantially planar shape. In the absence of an application of an external force on the medical implant 10, the medical implant 10 may be configured to automatically assume the closed configuration.

The tubular body 12 defines a longitudinally-extending passageway 14 therethrough dimensioned to receive a blood vessel, such as, for example, a vein of an AV fistula or a saphenous vein CABG. With a blood vessel, such as a saphenous vein, disposed within the passageway 14, the tubular body 12 is configured to exert a radially-inward force on the vein to restrict the outer diameter of the vein maximally to its venous physiological outer diameter while under venous or arterial pressure, or, restrict the outer diameter of the vein minimally to the outer diameter of a connecting artery. In the latter case, the tubular body 12 allows an isodiametric anastomotic connection. Intermediate levels of vein restriction between these extremes are also contemplated.

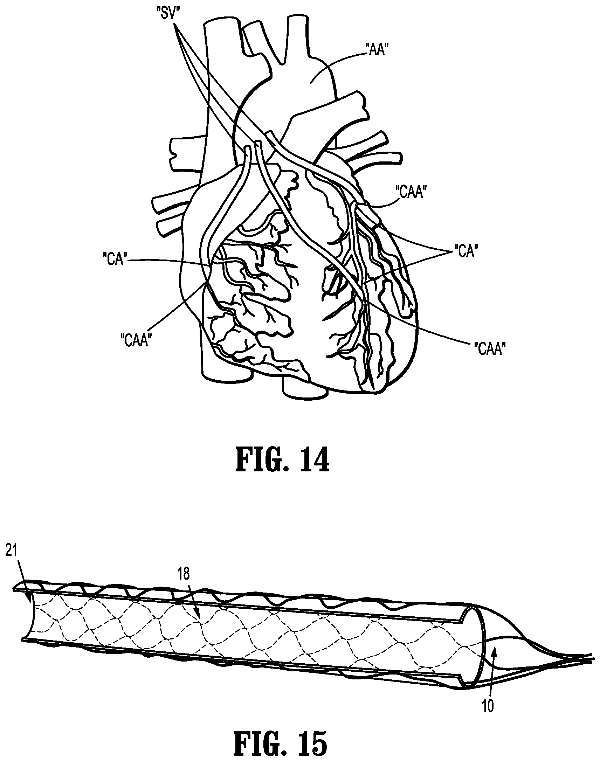

The tubular body 12 includes a proximal end portion 12a, a distal end portion 12b, and an intermediate portion 12c disposed between the proximal and distal end portions 12a, 12b. Each of the proximal and distal end portions 12a, 12b defines an opening 18, 20 in communication with the passageway 14 to allow for ends of a vein of an AV fistula to extend respectively therethrough or ends of a coronary artery bypass graft (see FIG. 14) to extend respectively therethrough. The proximal end portion 12a may have a flat proximal edge 22 that is perpendicular to a longitudinal axis of the passageway 14 and the distal end portion 12b may have a distal edge 24 that is also flat. In aspects, both the proximal and distal edges 22, 24 may be perpendicular or angled.

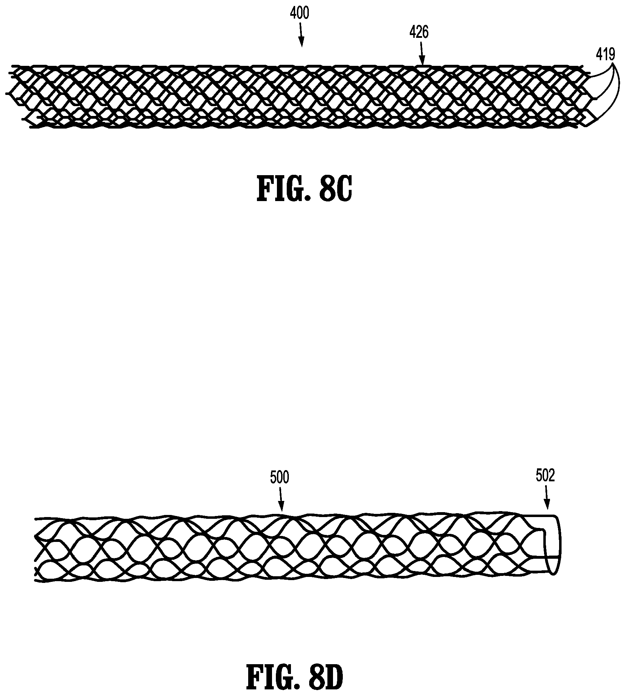

With brief reference to FIGS. 1B, 2, and 3B, a medical implant 10', similar to medical implant 10, is illustrated. Medical implant 10' differs from medical implant 10 by having a beveled end portion 12a' (e.g., a non-perpendicular relative to a longitudinal axis of the medical implant) and a flat end portion 12b'. The beveled end portion 12a' allows for a more physiological connection and resultant physiological fluid dynamics due to the shape of the blood vessels being supported. In one aspect, a medical implant 500 (FIG. 8D) includes an end having a solid ring 502 for suturing.

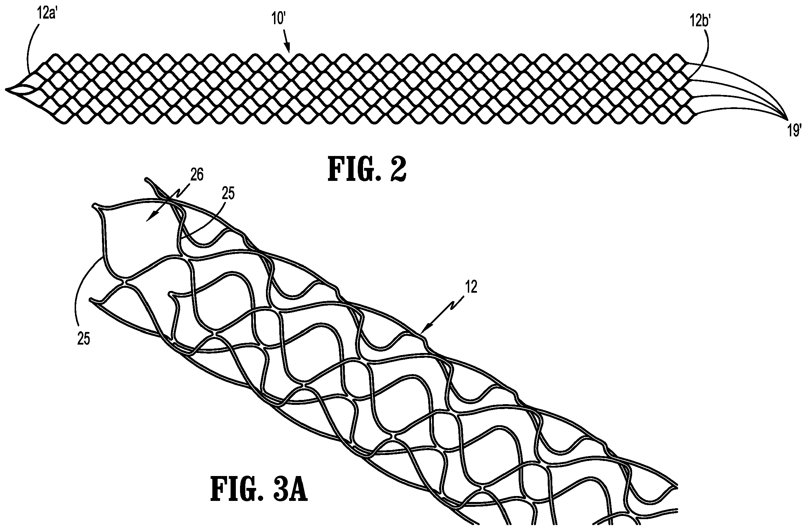

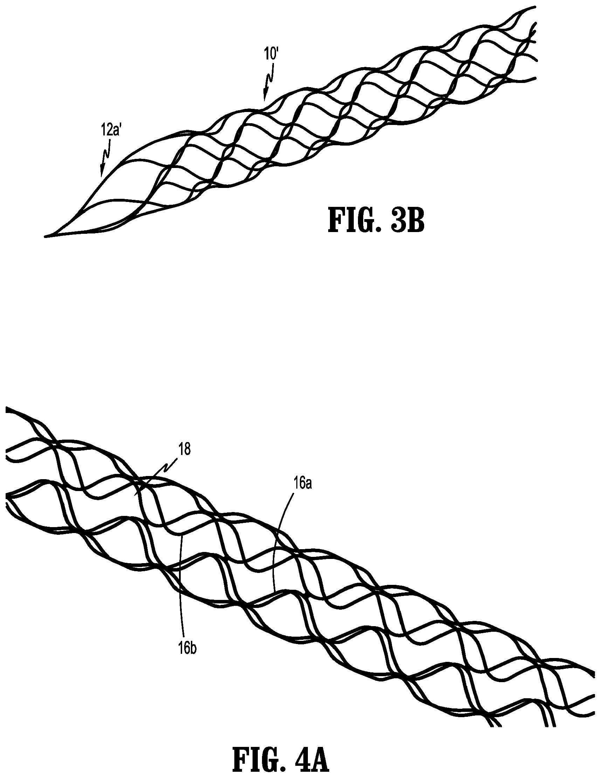

With reference to FIG. 3A, the tubular body 12 may include a plurality of undulating filaments or struts 25 that are connected to one another and extend along the length of the tubular body 12. Adjacent filaments 25 have opposing undulations such that the adjacent filaments 25 are monolithically formed with or otherwise connected at their respective peaks and valleys. The filaments 25 may have any suitable cross-sectional shape such as circular, oval, or polygonal (e.g., square or rectangular) and may have a cross-sectional width from about 100 µm to about 1,000 µm, which in certain aspect may be from about 200 µm to about 500 µm.

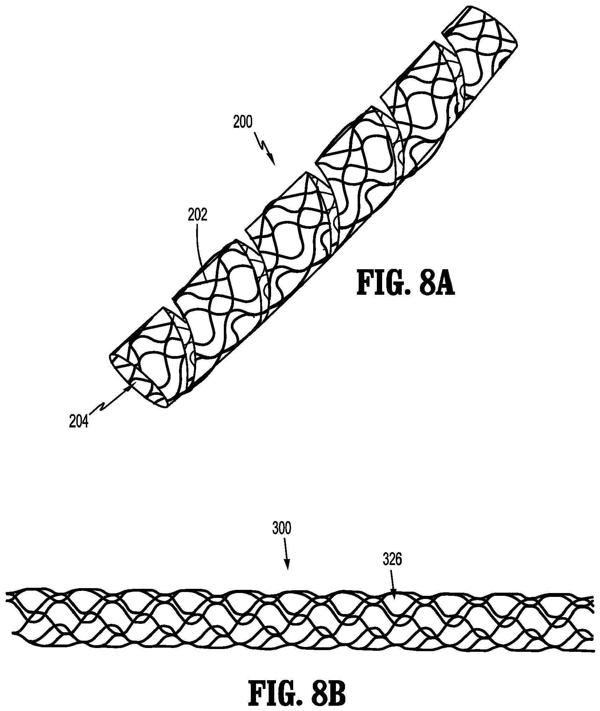

The connection between adjacent filaments 25 forms a plurality of discrete or enclosed cells 26 each having a 1:1 width to height ratio. In aspects, the width and height of each of the cells 26 may be from about 1 mm to about 5 mm, which in embodiments may be from about 2 mm to about 4 mm. Other dimensions and ratios for the cells 26 are also contemplated, such as the cells 326, 426 of the medical implants 300, 400 shown in FIGS. 8B and 8C, respectively. In aspects, the tubular body 12 may include a plurality of rows of cells, which may be from four rows 19' (as shown in FIG. 2) to eight rows 419 (as shown in FIG. 8C). As can be appreciated, for similarly sized medical implants, the cell size is inversely proportional to the number of rows, namely, the more rows of cells are included in the medical implant the smaller are the cells. The tubular body 12 may have a diameter (when disposed on the vessel) along its length of from about 3.0 mm to about 10.0 mm, and in some aspects from about 4.0 mm to about 8.0 mm. The tubular body 12 may have a length from about 5 cm to about 15 cm. In aspects, the tubular body 12 may be tapered along its length (e.g., conical) such that the proximal end portion 12a has a larger diameter than the distal end portion 12b or the distal end portion 12b has a larger diameter than the proximal end portion 12a. Other diameters and lengths of the tubular body 12 are also contemplated.

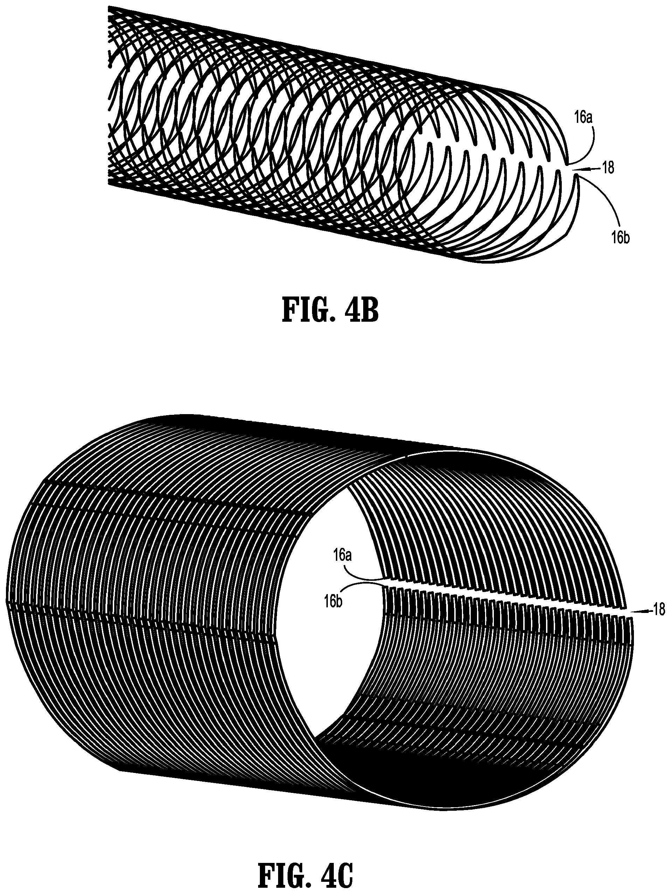

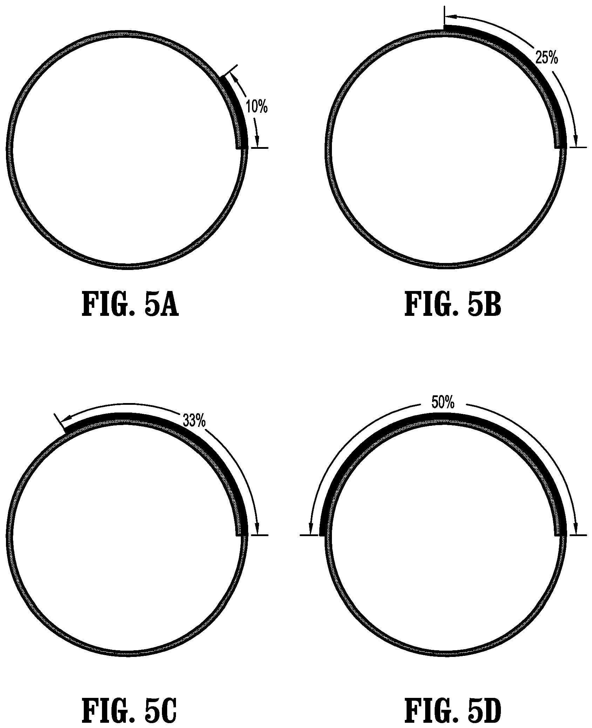

The tubular body 12 has a pair of longitudinal edges 16a, 16b (FIG. 4A) extending along the length of the tubular body 12. When the medical implant 10 is in its natural, closed configuration (FIG. 1A), the longitudinal edges 16a, 16b are disposed adjacent one another while being spaced to define a longitudinal seam 18 (FIGS. 4A-4C) along the length of the medical implant 10. In aspects, the longitudinal edges 16a, 16b may overlap one another from about 1 % to about 50%. In aspects, the longitudinal edges 16a, 16b may overlap one another about 10% (FIG. 5A), about 25% (FIG. 5B), about 33% (FIG. 5C), or about 50% (FIG. 5D).

With reference to FIGS. 6A, 6B, and 7A, another embodiment of a medical implant 100 is illustrated, similar to the medical implants of FIGS. 1A-4A. Since medical implant 100 is similar to the medical implant 10, only selected differences will be described in detail herein. The medical implant 100 includes a mesh-like tubular body 102 defining a longitudinally-extending passageway 104 configured to receive at least a venous portion of an AV fistula. The tubular body 102 defines a plurality of discrete or enclosed cells 106, the same or similar to cells 26, configured to receive respective ligated side branches of the venous portion of an AV fistula.

The tubular body 102 has a proximal end portion 102a, a distal end portion 102b, and an intermediate portion 102c disposed between the proximal and distal end portions 102a, 102b. The intermediate portion 102c defines a side opening 108 in communication with the passageway 104 and configured to receive and support an end of an arterial or venous portion of the AV fistula, as will be described in further detail below. The side opening 108 may have a circular shape, such as, for example, an oval (as shown in FIG. 6A), a circle (as shown in FIG. 6B), or the like, and has a diameter that is greater than a diameter of the cells 106. Other shapes for the side opening 108 are also contemplated. The opening 108 is formed by a looped filament 125 positioned in substantially opposing or diametrically opposing relation with a longitudinal seam 126 of tubular body 102.

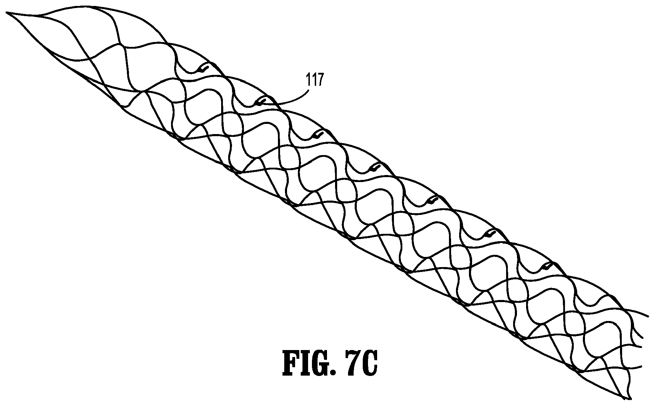

As shown in FIG. 7A, medical implant 10 or 10' may be integrally formed or otherwise coupled to medical implant 100 to form a single medical implant 150, in which the medical implant 100 forms a main tubular body disposed along a first axis, and the medical implant 10 or 10' forms a branching body disposed along a second axis that is transverse to the first axis. The angle between the first and second axes may be from about 10 degrees to about 170 degrees. In certain aspects, the medical implants 10 and 100 may be interconnected during implantation. In particular, the distal end portion 102b of the medical implant 10 may be connected, e.g., via sutures or adhesives, to the intermediate portion 102c of the medical implant 100 thereby interconnecting their respective passageways 14 and 104. In other aspects, the medical implants 10, 100 may be connected to one another utilizing fasteners that are affixed to the medical implants 10, 100, such as, for example, connectors 117, which are shown as hooks in FIG. 7C.

FIG. 7B illustrates a medical implant assembly 100' that forms a bifurcated Y- or T-shaped AV fistula and is configured to maintain and reinforce the angle of connection of the artery-vein anastomosis. The medical implant assembly 100' includes two medical implants or stents 102a', 102b' that present an obtuse angle "OA" for one side of the AV fistula and an acute angle "AA" for the other side of the AV fistula. The stents 102a', 102b' sit on either side of the connecting vessel of the AV fistula. To complete this AV fistula, the clinician first completes the anastomosis, then applies both the obtuse angle medical implant 102b' and the acute angle medical implant 102a'. The clinician may then apply stay sutures or a running suture along the connecting seam 118' defined between the halves 102a', 102b' of the medical implant assembly 100'.

For the medical implants of FIGS. 7A-7C, and all other implants here described, connectors 117 may be added that would be snapping/hooked together by the clinician, such as those shown in FIG. 7C. These connectors 117 may be added to the medical implants 10, 100, 200 and medial implant assembly 100' according to the present disclosure as shown in FIG. 7C, and would allow securing of connections and longitudinal seams.

With reference to FIG. 8A, yet another embodiment of a medical implant 200 is illustrated, similar to the medical implant 10 of FIGS. 1A-4A. Since medical implant 200 is similar to the medical implant 10, only selected differences will be described in detail herein. The medical implant 200 includes a mesh-like strip or ribbon 202 fabricated from a shape-memory material (e.g., nick-titanium), similar to the medical implants 10 and 100, that is resiliently biased toward a helical configuration. In the helical configuration, the medical implant 200 defines a longitudinally-extending passageway 204 configured to receive at least a venous portion of an AV fistula or a coronary artery bypass graft (e.g., a saphenous vein).

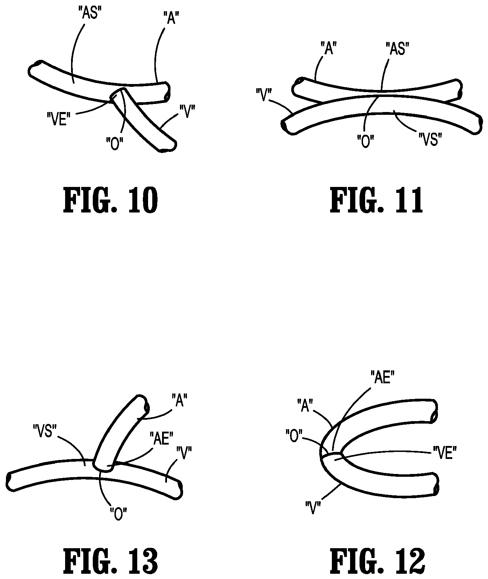

With reference to FIGS. 10-13, four types of methods for establishing an AV fistula are illustrated with each AV fistula utilizing one or more of the medical implants 10, 100, 150, 200 described above. The medical implants 10, 100, 150, 200 provide external support for the AV fistula to establish an AV fistula graft. During each of the four procedures, an anastomosis "O" or fluid connection is formed between a vein "V" and an artery "A." In particular, FIG. 10 illustrates an AV fistula formed by connecting an end "VE" of the vein "V" to a side "AS" of an artery "A"; FIG. 11 illustrates an AV fistula formed by connecting a side "VS" of the vein "V" to the side "AS" of the artery "A"; FIG. 12 illustrates an AV fistula formed by connecting the end "VE" of the vein "V" to an end "AE" of the artery "A"; and FIG. 13 illustrates an AV fistula formed by connecting the end "AE" of the artery "A" to the side "VS" of the vein "V."

The common initial step in each of the four procedures may include dissection and freeing up of the vein and artery segments identified for creation of the AV fistula. During this process, the vessels are typically minimally disturbed in their *in situ* position while ligating and cutting off any small interfering side branches. The flush removal of all side branches may be performed. Minimal trauma dissection and freeing up of the vessel segments prevents any damage to the intimal layers and subintimal layers of the vein or artery before application of the selected medical implant, where such damage may override one of the main functions of the medical implant of preventing intimal and subintimal damage to the delicate cellular and extracellular components of the vessel wall.

When performing the AV fistula of FIG. 10, the next step in the surgical procedure may include connecting the end "VE" of the vein "V" to the side "AS" of the artery "A" to form an anastomosis "O" therebetween. In certain aspects, the end "VE" of the vein "V" may be connected straight to, or on an angle with, the side "AS" of an artery "A". Here, only the medical implant 10 may be used, with a straight or angled end, and positioned over the vein "V" all the way to the anastomosis "O" site to provide external support to venous segment of the AV fistula.

More specifically before re-establishing blood flow/cross clamp release, the medical implant 10 is positioned over the vein "V." To position the medical implant 10 over the vein "V," the medical implant 10 may be transitioned toward the opened configuration by manually separating the longitudinal edges 16a, 16b of the medical implant 10 and then wrapping the medical implant 10 around the vein "V" by allowing the medical implant 10 to return to its unbiased, closed configuration about the vein "V." The medical implant 10 may have a length of about 10X the external diameter of the vein "V." In aspects, the clinician may trim the medical implant 10 to the appropriate length using a fine sharp surgical scissor. The thin struts of the medical implant 10 allow for such ease of trimming including the removal of any protruding partial struts.

In another aspect, the medical implant 10 may be positioned over the vein "V" by first inserting a spreading device, such as, for example, a C-shaped tube 21 (FIG. 15) into the medical implant 10 to expand the diameter of the medical implant 10. With the medical implant 10 expanded beyond its natural, closed configuration, the C-shaped tube 21 and the medical implant 10 are slid over the vein "V." The C-shaped tube 21 is then removed from the medical implant 10, thereby allowing the medical implant 10 to return to its unbiased, closed configuration about the vein "V."

When positioning the medical implant 10 over the vein "V," each of the side branch ligatures of the vein "V" is aligned with a respective cell 26 of the medical implant 10 to prevent/minimize luminal encroachment due to ligature and side branch tissue being pinned between the tubular body 12 and the vein "V." With the medical implant 10 disposed about the vein "V," the distal end portion 12b of the medical implant 10 is directly coupled to the side "AS" of the artery "A" to cover the anastomosis "O" site and fix the medical implant 10 in place. When directly connecting the medical implant 10 to the artery "A," the distal end portion 12b of the medical implant 10 may be sutured to the artery "A." Other mechanisms for fastening the medical implant 10 to the artery "A" are contemplated.

With the medical implant 10 in place, blood flow may be re-established, whereby the vein "V" is permitted by the medical implant 10 to expand radially-outward. The medical implant 10 exerts a threshold force oriented in a radially-inward direction to limit the amount of radial expansion of the vein "V."

When forming the AV fistula of FIG. 11, with the side "VS" of the vein "V" and the side "AS" of the artery "A" fluidly connected to one another, the medical implant 10 is positioned over the vein "V," the artery "A," and a large, elongated oval-shaped anastomosis "O" to provide external support to the AV fistula. Here, a larger diameter version of the medical implant 10 is positioned over the entire region of the anastomosis "O" to provide external support to the attached arterial and venous segment of the AV fistula. In this case the diameter of the medical implant 10 is approximated to have the combined diameters of the artery "A" and vein "V" at the midpoint of the anastomosis "O."

When forming the AV fistula of FIG. 12, a straight end-to-end anastomosis "O' is performed by connecting the end "AE" of an artery "A" to the end "VE" of a vein "V". The medical implant 10, with a diameter range between the physiological outer diameter of the vein "V" and the physiological outer diameter of the artery "A," may be then carefully added over the vein "V" and extended over the anastomosis "O" to provide external support to both the vein "V" and a small portion of the artery "A" to complete this type of AV fistula.

When forming the AV fistula of FIG. 13, which involves connecting the straight or angled end "AE" of the artery "A" to the side "VS" of the vein "V," the medical implant 100 of FIGS. 6A or 6B is selected such that the round or oval opening 108 closely matches the projected geometry of the anastomosis "O." The straight or angled end of the artery "A" is firstly gently inserted through the round or oval opening 108, and the medical implant 100 is pushed back along the artery "A" so that it will allow the next step of completing the anastomosis "O." When the anastomosis "O" is complete, the medical implant 100 is slid back down the artery "A" and opened along the longitudinal seam 18 and wrapped around the vein "V." Optional 'stay sutures' can be added to the anastomosis "O" to further secure and reinforce the medical implant 100 in this region in this type of AF fistula.

Another method of utilizing the medical implant 100 of FIGS. 6A or 6B for the AV fistula of FIG. 13 includes positioning the medical implant 100 over the vein "V" and aligning the side opening 108 of the medical implant 100 with the site on the vein "V" selected for connection with the artery "A." The medical implant 100 may be wrapped around the outer surface of the vein "V" in a similar manner as that described with reference to the procedure of FIG. 10. The end "AE" of the artery "A" is passed through or brought adjacent to the side opening 108 of the medical implant 100 and joined by anastomosis "O" to the side "VS" of the vein "V," thereby forming a fluid connection therebetween. With the artery "A" joined to the vein "V," the medical implant 100 covers and externally supports the anastomosis site "O," the vein "V," and the end "AE" of the artery "A."

As shown in FIG. 7A, another medical implant, such as medical implant 10, may be used to cover the artery "A" of the AV fistula of FIG. 13. The distal end portion 12b of the medical implant 10 may be joined to the side of the medical implant 100, whereby the assembly of the medical implants 10, 100 forms the single medical implant 150, which provides external support to both the vein "V" and artery "A." The medical implant 150 completely covers and reinforces the artery and venous portions of the AV fistula of types in FIGS. 10 and 13 and where the connecting portions of the implants utilize wider/stronger struts to help maintain the angle of connection.

In aspects, medical implant 200 may be utilized instead of or in addition to medical implants 10, 100 to cover any of the AV fistulas described above by wrapping, in a helical manner, the medical implant 200 about the venous portion "V" and/or arterial portion "A" of the AV fistula.

With reference to FIG. 14, another surgical procedure is proposed utilizing one or more of the medical implants 10, 100, and/or 200 described above. The surgical procedure is a coronary artery bypass graft ("CABG"), which may include applying the medical implant 10 (FIG. 1A) to a saphenous vein "SV" following the vein excision for CABG revascularization surgery.

In one embodiment, an initial step in the surgical procedure involves dissection and freeing of a saphenous vein "SV." The saphenous vein "SV" may be dissected free in standard fashion, leaving it in situ while ligating and cutting off all side branches. Since side-branch ligations that leave behind excessive tissue "stumps" may create sites of significant luminal encroachments when the medical implant is applied, the flush removal of all side branches is preferred, as noted above with respect to dissecting the vein used for an AV fistula. The second step involves testing the excised vein "SV" for potential leaks. The isolated saphenous vein "SV" may be tested by injecting cold heparinized blood or appropriate physiological buffer between 2-37 degrees Celsius to inflate the vein "SV" with a syringe from one end while the other end is occluded. Such inflation of the vein "SV" using a standard syringe can create extreme non-physiological pressures, for example, greater than about 350 mmHg, and is often a main cause of traumatic damage to the vein wall. Therefore, a modified pressure-limiting syringe may be used - one that limits the inflation pressure to a level suitable for detecting leaks, but not sufficient to cause tissue stretch damage. In embodiments, a pressure-limiting syringe that allows up to about 15 mmHg pressure, a typical physiological pressure for veins, may be used. Using this approach, observed leaks in the vein wall are readily identified and safely repaired, and the vein "SV" remains free of injury due to excessive pressurization. Lower pressure leak testing avoids damage to the intimal and subintimal layers in the vein "SV" which can occur without precaution during vein harvesting even before application of the medical implant 10. Such damage during leak testing would potentially override one of the main functions of the medical implant 10, which is to prevent intimal and subintimal damage to the vein "SV."

The next step involves assembling the harvested vein segment and the medical implant 10, which may be done before or after suturing the vein "SV" to a coronary artery "CA" and an aortic arch "AA" of the patient. During this step, with appropriate cross clamps in place to prevent blood flow into the vein "SV," the medical implant 10 may be gently opened along the seam 18 with a clinician's fingers, and the vein "SV" is placed within the passageway 14 of the medical implant 10. The medical implant 10 will naturally fold around the vein graft "SV" upon release of the opened seam 18. Here, care may be taken at each site of a vein side branch to align the side branch ligature with the nearest pore (open cell) 26 of the medical implant 10. This prevents and/or minimizes luminal encroachment due to ligature and side branch tissue being pinned between a stent strut of the medical implant 10 and the vein "SV." Alternatively, opening the medical implant 10 may be assisted with the C-shaped tube 21 (FIG. 15), which slides along the seam 18 of the medical implant 10, or a similar long straw is pre-positioned within the entire length of the medical implant 10, thereby forcing the seam 18 to open widely allowing the vein "SV" to be dropped into a lumen of the C-shaped tube 21.

The C-shaped tube 21 is then removed axially, leaving the medical implant 10 and vein "SV" in contact to form the externally-supported venous graft. Care may be taken that the medical implant 10 externally supports/covers the entire vein graft from the aortic arch "AA" to the coronary artery anastomosis "CAA" (leaving from about 0 mm to about 1 mm gap of non-externally-supported vein "SV"). At this point, with the anastomoses complete and the medical implant 10 in place, cross clamps may be removed, and the vein "SV" is then inflated under arterial blood pressures of about 80-120 mmHg, causing the vein "SV" to contact the inner annular surface of the medical implant 10. Optional anchoring sutures may be added at the anastomoses and elsewhere to help fix the medical implant 10 in place. Any of the components described herein may be fabricated from either metals, plastics, resins, composites or the like taking into consideration strength, durability, wearability, weight, resistance to corrosion, ease of manufacturing, cost of manufacturing, and the like.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

## Claims

1. A medical implant (100) for providing external support to an arteriovenous fistula , the medical implant comprising:
a mesh-like main tubular body (102) configured to transition between an opened configuration
and a closed configuration, in which the main tubular body defines a longitudinally-extending main passageway (106) therethrough configured to receive a first blood vessel, the main tubular body being resiliently biased toward the closed configuration, the main tubular body including:
a proximal end portion (102a) defining an opening in communication with the main passageway;
a distal end portion (102b) defining an opening in communication with the main passageway; and
an intermediate portion (102c) disposed between the proximal end portion (102a) and the distal end portion (102b), wherein the intermediate portion defines a side opening (108) in communication with the main passageway and configured to receive and support a second blood vessel therein, wherein the main tubular body has a pair of longitudinal edges configured to be disposed adjacent one another when the main tubular body is in the closed configuration, the pair of longitudinal edges together defining a seam (126) that extends along a length of the main tubular body, the seam and the side opening being disposed in opposing relation to one another; and the opening of the intermediate portion (102c) being formed by a looped filament (125) positioned in substantially opposing or diametrically opposing relation with the longitudinal seam (126) of the main tubular body (102).

2. The medical implant according to claim 1, wherein the side opening (108) has a circular shape.

3. The medical implant according to claim 1 or claim 2, wherein the distal end portion of the main tubular body (102) has a distal edge extending at a non-perpendicular angle relative to a longitudinal axis of the main tubular body.

4. The medical implant according to any preceding claim, further comprising:
a branching tubular body configured to transition between an opened configuration and a closed configuration, in which the branching tubular body defines a longitudinally-extending branching passageway therethrough, the branching passageway is in communication with the main passageway and is configured to receive and support the second blood vessel, the branching tubular body being resiliently biased toward the closed configuration, the branching tubular body including:
a branching proximal end portion defining an opening in communication with the branching passageway; and
a branching distal end portion defining an opening in communication with the branching passageway.

5. The medical implant according to claim 4, wherein the main tubular body and the branching tubular body are fabricated from a mesh that defines a plurality of discrete cells, each of the discrete cells having a smaller diameter than the side opening.

6. The medical implant according to claim 4 or claim 5, wherein the main tubular body and the branching tubular body are fabricated from a shape memory material.

## Patentansprüche

1. Medizinisches Implantat (100) zum Bereitstellen externer Unterstützung für eine arteriovenöse Fistel, das medizinische Implantat umfassend:
einen maschenartigen rohrförmigen Hauptkörper (102), der konfiguriert ist, um zwischen einer geöffneten Konfiguration und einer geschlossenen Konfiguration zu wechseln, wobei der rohrförmige Hauptkörper einen sich in Längsrichtung dort hindurch erstreckenden Hauptdurchgang (106) definiert, der konfiguriert ist, um ein erstes Blutgefäß aufzunehmen, wobei der rohrförmige Hauptkörper zu der geschlossenen Konfiguration hin elastisch vorgespannt ist, wobei der rohrförmige Hauptkörper einschließt:
einen proximalen Endabschnitt (102a), der eine Öffnung in Kommunikation mit dem Hauptdurchgang definiert;
einen distalen Endabschnitt (102b), der eine Öffnung in Kommunikation mit dem Hauptdurchgang definiert; und
einen Zwischenabschnitt (102c), der zwischen dem proximalen Endabschnitt (102a) und dem distalen Endabschnitt (102b) angeordnet ist, wobei der Zwischenabschnitt eine seitliche Öffnung (108) in Kommunikation mit dem Hauptdurchgang definiert und konfiguriert ist, um ein zweites Blutgefäß darin aufzunehmen und zu stützen, wobei der rohrförmige Hauptkörper ein Paar Längskanten aufweist, die konfiguriert sind, um aneinander angrenzend angeordnet zu sein, wenn sich der rohrförmige Hauptkörper in der geschlossenen Konfiguration befindet, wobei das Paar von Längskanten zusammen eine Naht (126) definieren, die sich entlang einer Länge des rohrförmigen Hauptkörpers erstreckt, wobei die Naht und die seitliche Öffnung in einem entgegengesetzten Verhältnis zueinander angeordnet sind; und
wobei die Öffnung des Zwischenabschnitts (102c) durch ein schlaufenförmiges Filament (125) gebildet wird, das im Wesentlichen in gegenüberliegender oder diametral gegenüberliegender Beziehung zu der Längsnaht (126) des rohrförmigen Hauptkörpers (102) positioniert ist.

2. Medizinisches Implantat nach Anspruch 1, wobei die seitliche Öffnung (108) eine kreisförmige Form aufweist.

3. Medizinisches Implantat nach Anspruch 1 oder 2, wobei der distale Endabschnitt des rohrförmigen Hauptkörpers (102) eine distale Kante aufweist, die sich in einem nicht senkrechten Winkel relativ zu einer Längsachse des rohrförmigen Hauptkörpers erstreckt.

4. Medizinisches Implantat nach einem der vorstehenden Ansprüche, ferner umfassend:
einen abzweigenden rohrförmigen Körper, der konfiguriert ist, um zwischen einer geöffneten Konfiguration und einer geschlossenen Konfiguration zu wechseln, wobei der abzweigende rohrförmige Körper einen sich in Längsrichtung dort hindurch erstreckenden abzweigenden Durchgang definiert, wobei der abzweigende Durchgang in Kommunikation mit dem Hauptdurchgang ist und konfiguriert ist, um das zweite Blutgefäß aufzunehmen und zu stützen, wobei der abzweigende rohrförmige Körper zu der geschlossenen Konfiguration hin elastisch vorgespannt ist, wobei der abzweigende rohrförmige Körper einschließt:
einen abzweigenden proximalen Endabschnitt, der eine Öffnung in Kommunikation mit dem abzweigenden Durchgang definiert; und
einen abzweigenden distalen Endabschnitt, der eine Öffnung in Kommunikation mit dem abzweigenden Durchgang definiert.

5. Medizinisches Implantat nach Anspruch 4, wobei der rohrförmige Hauptkörper und der abzweigende rohrförmige Körper aus einem Netz hergestellt sind, das eine Vielzahl von diskreten Zellen definiert, wobei jede der diskreten Zellen einen kleineren Durchmesser als die seitliche Öffnung aufweist.

6. Medizinisches Implantat nach Anspruch 4 oder 5, wobei der rohrförmige Hauptkörper und der abzweigende rohrförmige Körper aus einem Formgedächtnismaterial hergestellt sind.

## Revendications

1. Implant médical (100) permettant de fournir un support externe à une fistule artérioveineuse, l'implant médical comprenant :
un corps tubulaire principal de type maillage (102) conçu pour effectuer une transition entre une configuration ouverte et une configuration fermée, le corps tubulaire principal définissant une voie de passage principale s'étendant longitudinalement (106) à travers celui-ci, conçue pour recevoir un premier vaisseau sanguin, le corps tubulaire principal étant élastiquement sollicité en direction de la configuration fermée, le corps tubulaire principal comportant :
une partie d'extrémité proximale (102a) définissant une ouverture en communication avec la voie de passage principale ;
une partie d'extrémité distale (102b) définissant une ouverture en communication avec la voie de passage principale ; et
une partie intermédiaire (102c) disposée entre la partie d'extrémité proximale (102a) et la partie d'extrémité distale (102b), la partie intermédiaire définissant une ouverture latérale (108) en communication avec la voie de passage principale et conçue pour recevoir et supporter un second vaisseau sanguin en son sein, le corps tubulaire principal ayant une paire de bords longitudinaux conçus pour être disposés l'un à côté de l'autre lorsque le corps tubulaire principal est dans la configuration fermée, la paire de bords longitudinaux définissant ensemble une jointure (126) qui s'étend le long d'une longueur du corps tubulaire principal, la jointure et l'ouverture latérale étant disposées en relation opposée l'une par rapport à l'autre ; et
l'ouverture de la partie intermédiaire (102c) étant formée par un filament bouclé (125) positionné dans une relation sensiblement opposée ou diamétralement opposée par rapport à la jointure longitudinale (126) du corps tubulaire principal (102).

2. Implant médical selon la revendication 1, dans lequel l'ouverture latérale (108) a une forme circulaire.

3. Implant médical selon la revendication 1 ou la revendication 2, dans lequel la partie d'extrémité distale du corps tubulaire principal (102) a un bord distal s'étendant selon un angle non perpendiculaire par rapport à un axe longitudinal du corps tubulaire principal.

4. Implant médical selon l'une quelconque revendication précédente, comprenant en outre :
un corps tubulaire de ramification conçu pour effectuer une transition entre une configuration ouverte et une configuration fermée, le corps tubulaire de ramification définissant une voie de passage de ramification s'étendant longitudinalement à travers celui-ci, la voie de passage de ramification étant en communication avec la voie de passage principale et étant conçue pour recevoir et supporter le second vaisseau sanguin, le corps tubulaire de ramification étant élastiquement sollicité en direction de la configuration fermée, le corps tubulaire de ramification comportant :
une partie d'extrémité proximale de ramification définissant une ouverture en communication avec la voie de passage de ramification ; et
une partie d'extrémité distale de ramification définissant une ouverture en communication avec la voie de passage de ramification.

5. Implant médical selon la revendication 4, dans lequel le corps tubulaire principal et le corps tubulaire de ramification sont fabriqués à partir d'un maillage qui définit une pluralité de cellules distinctes, chacune des cellules distinctes ayant un diamètre plus petit que l'ouverture latérale.

6. Implant médical selon la revendication 4 ou la revendication 5, dans lequel le corps tubulaire principal et le corps tubulaire de ramification sont fabriqués à partir d'un matériau à mémoire de forme.
